(19) Europäisches Patentamt
European Patent Office
Office européen des brevets

(11) EP 1 459 801 B1

(12) # FASCICULE DE BREVET EUROPEEN

(45) Date de publication et mention de la délivrance du brevet:
**31.07.2013 Bulletin 2013/31**

(51) Int Cl.:
***A61K 8/06*** *(2006.01)* ***A61K 8/04*** *(2006.01)*
***A61K 8/81*** *(2006.01)* ***A61Q 17/04*** *(2006.01)*

(21) Numéro de dépôt: **04300133.8**

(22) Date de dépôt: **11.03.2004**

(54) **Emulsions notamment solaires de type eau-dans-huile, et leur procédé de préparation**

Wasser-in-Öl Emulsionen, insbesondere für Sonnenschutz, und Verfahren zu deren Herstellung

Water-in-oil emulsions, especially for suncare, and process for preparing them

(84) Etats contractants désignés:
**AT BE BG CH CY CZ DE DK EE ES FI FR GB GR HU IE IT LI LU MC NL PL PT RO SE SI SK TR**

(30) Priorité: **14.03.2003 FR 0303157**
**25.09.2003 FR 0311263**

(43) Date de publication de la demande:
**22.09.2004 Bulletin 2004/39**

(73) Titulaire: **SOCIETE D'EXPLOITATION DE PRODUITS POUR LES INDUSTRIES CHIMIQUES-SEPPIC**
**F-75321 Paris Cédex 07 (FR)**

(72) Inventeurs:
- **Amalric, Chantal**
  **81700 Blan (FR)**
- **Roso, Alicia**
  **81710 Saix (FR)**
- **Tabacchi, Guy**
  **75007 Paris (FR)**

(74) Mandataire: **Conan, Philippe Claude et al**
**L'Air Liquide SA,**
**75 Quai d'Orsay**
**75321 Paris Cedex 07 (FR)**

(56) Documents cités:
**EP-A- 0 503 853** **EP-A- 1 325 729**
**WO-A-02/100374** **FR-A- 2 816 836**

- **R. PONS ET AL.: "novel preparation methods for highly concentrated water-in oil emulsions" COLLOIDS AND SURFACES A: PHYSICOCHEMICAL AND ENGINEERING ASPECTS, vol. 91, 1994, pages 259-266, XP008026972**

Remarques:
Le dossier contient des informations techniques présentées postérieurement au dépôt de la demande et ne figurant pas dans le présent fascicule.

EP 1 459 801 B1

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

La présente invention a pour objet des émulsions de type eau dans huile, à forte teneur en phase aqueuse, leur procédé de préparation et leurs utilisations.

Arrière-plan technologique

Les émulsions permettent de véhiculer à la fois des substances hydrosolubles et liposolubles et trouvent donc notamment application dans les domaines cosmétique, pharmaceutique et vétérinaire, et dans le domaine des détergents.

Dans le domaine cosmétique, il existe une exigence de la part de l'utilisateur de produits sous forme d'émulsions de disposer d'émulsions qui présentent des caractéristiques sensorielles appropriées. Les émulsions qui procurent une sensation de fraîcheur et qui sont ressenties en application sur la peau comme non "collantes" sont particulièrement recherchées.

Les émulsions sont classifiées selon la nature de la phase continue (également appelée "phase externe"), dans laquelle sont dispersées des gouttelettes de l'autre phase (dite "phase interne").

Dans le cas où les gouttelettes d'huile sont dispersées dans une phase continue aqueuse, on nomme le système une émulsion de type "huile dans eau" (H/E).

Dans le cas où les gouttelettes d'eau sont dispersées dans une phase continue huileuse, l'émulsion est de type "eau dans huile" (E/H).

De façon générale, parmi ces deux types d'émulsions, il est plus facile de fabriquer des émulsions de type huile dans eau (H/E), car des émulsions de type E/H sont intrinsèquement instables sur le plan thermodynamique. En effet, si on mélange des quantités égales d'eau et d'huile, on observe toujours la formation d'une émulsion à phase continue aqueuse, car les forces de cohésion entre des molécules d'eau sont plus fortes que celles entre des molécules d'huile.

Les émulsions à phase continue huileuse (E/H) présentent néanmoins de nombreux intérêts:

- la séparation entre les gouttelettes d'eau réduit la possibilité de prolifération de micro-organismes. L'utilisation d'antiseptiques, essentielle lorsque la phase continue est aqueuse, peut être évitée ;
- elles se conservent bien à basse température, étant bien moins sensibles à cet égard que des émulsions de type H/E ;
- la phase continue huileuse recouvre la peau et la protège de la déshydratation et contre des substances externes.

Dans la mise au point d'un système d'émulsion à phase continue huileuse, deux types de modifications ont été envisagés:

- des modifications d'ordre mécanique concernant la combinaison des phases (ordre d'ajout des phases, contrôle du débit pendant la combinaison des phases, température des phases, vitesse d'agitation etc.) ;
- des modifications au niveau des constituants chimiques ayant pour résultat la stabilisation de l'émulsion.

En ce qui concerne les modifications d'ordre mécanique, les protocoles opératoires mis en oeuvre pour préparer des émulsions E/H nécessitent généralement:

a) un apport énergétique important, sous forme d'activation thermique (les phases aqueuses et grasses sont typiquement chauffées à 80°C), ce qui doit parfois être suivi d'un refroidissement progressif bien maîtrisé; et/ou
b) la création de turbulences dans le milieu biphasique à émulsionner (vitesse d'agitation élevée (milliers de tours par minute) et cisaillement important amené par des géométries spécifiques des agitateurs).

En ce qui concerne les modifications d'ordre chimique, on peut citer:

a) l'utilisation de cires microcristallines, telles que l'ozokérite, qui absorbent l'huile et empêchent son exsudation ;
b) l'utilisation de paraffines liquides en tant que phase grasse, car celles-ci sont plus faciles à émulsifier ;
c) l'ajout de sels minéraux tels que notamment le chlorure de sodium ou le chlorure de magnésium, permettant d'augmenter la cohésion du film interfacial.

Le brevet US-5746945 décrit des émulsions de type eau dans huile qui sont stabilisées par un système émulsifiant ayant deux composants: a) un copolymère polysiloxane polyalkyl polyéther et b) un dérivé de l'anhydride phtalique (un monoamide). La méthode de préparation d'émulsions dans ce document consiste en l'ajout progressif de la phase aqueuse à la phase huileuse. Les deux phases sont chacune indépendamment de l'autre chauffées à 160 à 165°F (71

à 74°C) avant d'être combinées pour obtenir l'émulsion.

Le document WO-97/40814 décrit des émulsions E/H destinées notamment à être utilisées pour imprégner des lingettes pour bébé. La phase organique des émulsions contient nécessairement une cire. Les émulsionnants utilisés sont de type acide carboxylique, substitués par des hydrocarbures, ou des copolymères "block" dBA, impliquant des monomères tels que l'acide 12-hydroxystéarique et l'éthylène glycol, ou un alkyldiméthicone copolyol. En ce qui concerne le procédé utilisé pour préparer les émulsions, on chauffe typiquement la phase grasse et la phase aqueuse à 160°F (71°C) puis on les mélange à cette température pour obtenir l'émulsion.

WO02/100374 décrit une émulsion huile-dans-eau contenant 86.3% d'eau, 3.7% d'un polyacrylate. Le polyacrylate est un gélifiant de la phase aqueuse.

EP0503853 décrit un épaississant ou gélifiant comprenant un polymère soluble dans l'eau ainsi que la préparation d'une émulsion eau-dans-huile.

FR2816836 décrit une composition de bronzage formulée sous la forme d'une composition huile-dans-l'eau ou eau-dans-huile.

EP1325729 décrit une composition contenant un copolymère siliconé et un polymère d'AMPS.

R. Pons et al. décrit une émulsion eau-dans-huile ainsi que les microémulsions correspondantes.

EP1243252 décrit une émulsion eau-dans-huile avec du Sepigel 305 et des émulsifiants. EP1166770 décrit une émulsion eau-dans-huile avec un copolymère d'AMPS et du palmitate de dextrine.

EP1000542 décrit des émulsions eau-dans-huile avec du Sepigel 305 et des émulsifiants.

Les procédures typiques de l'art antérieur présentent donc un certain nombre d'inconvénients, liés notamment à la nécessité de fournir un apport énergétique important pour réaliser l'émulsion.

Un problème à résoudre consiste donc à fournir des émulsions E/H à forte teneur en phase aqueuse, qui présentent en particulier une texture fraîche et non collante.

Un autre problème à résoudre consiste à disposer d'un procédé de préparation de telles émulsions, qui soit :

- simple, c'est-à-dire que le nombre d'étapes mises en oeuvre soit réduit et que les facteurs tels que le contrôle exact des débits d'introduction des phases ne soient pas critiques pour le bon fonctionnement du procédé, et
- économique, c'est-à-dire qu'il ne nécessite pas de dépenser beaucoup d'énergie ni pour chauffer les phases à combiner ni dans l'agitation vigoureuse à fournir lors de la préparation de l'émulsion.

<u>Résumé de l'invention</u>

Il a maintenant été découvert, et c'est le fondement de la présente invention, qu'en ajoutant une phase huileuse à une phase aqueuse gélifiée, il est possible d'obtenir des émulsions E/H ayant les caractéristiques précitées, sans apport thermique et mécanique important.

Selon un premier aspect, la présente demande a donc pour objet une émulsion comme mentionnée à la revendication 1 constituée d'une phase externe huileuse et d'une phase aqueuse gélifiée, ladite phase aqueuse représentant de 60 à 98% en poids, de préférence de 80 à 98% en poids, de la composition, caractérisée en ce que :

- la phase aqueuse comprend un polymère de type polyélectrolyte dont les sites ioniques sont associés à leurs contre-ions,
  et
- la phase huileuse comprend une ou plusieurs huiles et un système émulsionnant à caractère lipophile comprenant un ou plusieurs tensioactifs émulsionnants.

Selon un second aspect, la présente demande a pour objet un procédé de préparation d'une émulsion de type eau dans huile à forte teneur en phase aqueuse comme mentionnée à la revendication 1 comprenant les étapes suivantes :

a) on prépare une phase grasse comprenant une ou plusieurs huiles en présence d'un système émulsionnant à caractère lipophile comprenant un ou plusieurs tensioactifs émulsionnants;
b) on prépare, indépendamment de la phase grasse, une phase aqueuse gélifiée contenant un polymère de type polyélectrolyte;
c) on ajoute la phase grasse sur la phase aqueuse.

Selon un troisième aspect, la présente demande a pour objet des préparations cosmétiques, pharmaceutiques, vétérinaires ou détergentes contenant une émulsion telle que définie ci-dessus.

Description détaillée de la présente invention

Conformément au procédé de l'invention, on prépare dans un premier temps une phase grasse comprenant une ou plusieurs huiles choisies notamment parmi :

- les huiles d'origine végétale, telles que l'huile d'amandes douces, l'huile de coprah, l'huile de monoï, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula ;
- les huiles végétales et leurs esters méthyliques éthoxylés ;
- les huiles d'origine animale, telles que le squalène, le squalane ;
- les huiles minérales, telles que l'huile de paraffine, l'huile de vaseline et les isoparaffines ;
- les huiles synthétiques, notamment les esters d'acides gras tels que le myristate de butyle, le myristate de propyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les monoglycérides, diglycérides et triglycérides d'acides gras comme le triheptanoate de glycérol, les alkylbenzoates, les polyalphaoléfines, les polyoléfines comme le polyisobutène, les isoalcanes de synthèse comme l'isohexadecane, l'isododécane et les huiles perfluorées. Les huiles de silicone sont également susceptibles d'être utilisées dans le cadre de la présente invention. Parmi ces dernières, on peut plus particulièrement citer les diméthylpolysiloxanes, les méthylphénylpolysiloxanes, les silicones modifiés par des amines, les silicones modifiés par des acides gras, les silicones modifiés par des alcools, les silicones modifiés par des alcools et des acides gras, des silicones modifiés par des groupements polyéther, des silicones époxy modifiés, des silicones modifiés par des groupements fluorés, des silicones cycliques et des silicones modifiés par des groupements alkyles. Toutefois, pour des raisons pratiques, il peut être souhaitable que la phase grasse ne comprenne pas d'huile de silicone.

L'émulsion eau-dans-huile comprend généralement de 2 à 40 % en poids, de préférence de 2 à 20% en poids, d'huile(s).

La phase grasse est préparée en présence d'un système émulsionnant à caractère lipophile, comprenant un ou plusieurs tensioactifs émulsionnants.

Parmi les tensioactifs émulsionnants susceptibles d'être utilisés dans le cadre de la présente invention, on citera notamment les lipoaminoacides et leurs sels ; les lipopeptides et leurs sels; les esters de sorbitan comme par exemple le produit commercialisé sous la dénomination MONTANE® 80 par la société SEPPIC; les esters de polyglycérol comme par exemple les produits commercialisés sous la dénomination ISOLAN® GI34 par BASF et PLUROL® DIISOSTEARIQUE par GATTEFOSSE ; l'huile de ricin éthoxylée et l'huile de ricin hydrogénée éthoxylée comme par exemple le produit commercialisé sous la dénomination SIMULSOL® 989 par la société SEPPIC ; le stéarate de glycérol ; les polyhydroxystéarates de polyglycol ou de polyglycérol comme par exemple les produits dénommés HYPERMER® B246, ARLACEL® P135 commercialisés par la société UNIQEMA, le produit dénommé DEHYMULS® PGPH commercialisé par la société COGNIS, le produit dénommé DECAGLYN® 5HS commercialisé par la société NIKKO ; les copolymères polyéthylèneglycol-alkylglycol comme le PEG-45 dodécylglycol copolymère tel que le produit commercialisé sous la dénomination ELFACOS ST 9® par la société AKZO, les esters de sorbitan éthoxylés comme par exemple les produits commercialisés sous la dénomination MONTANOX® par la société SEPPIC ; les acylats de protéines faiblement éthoxylés (de 1 à 3 groupements OE) ; la cire d'abeille éthoxylée comme par exemple le produit dénommé APIFIL® commercialisé par la société GATTEFOSSE; les émulsionnants cationiques comme les aminoxydes, le quaternium 82 et les tensio-actifs décrits dans la demande de brevet WO 96/00719 et principalement ceux dont la chaîne grasse comprend au moins 16 atomes de carbone ; les esters de sucrose, les esters de méthylglucoside ethoxylés ou non ; les acides gras éthoxylés ; les alcools gras éthoxylés ; les émulsionnants anioniques comme le décylphosphate ou le cétéarylsulfate ; le polyoxystéarate d'aluminium, tel que par exemple le produit commercialisé sous la dénomination MANALOX® par la société RHODIA ; le stéarate de magnésium ; le stéarate d'aluminium.

Des tensioactifs émulsionnants siliconés non-ioniques et anioniques sont également susceptibles d'être utilisés dans le cadre de la présente invention, même si pour des raisons pratiques (ils peuvent entraîner une modification des propriétés sensorielles des émulsions obtenues), ils ne représentent pas un aspect préféré de l'invention.

Il est également possible d'utiliser des tensioactifs émulsionnants de type alkylpolyglycoside, par exemple ceux décrits dans la demande de brevet FR-A-2 790 977, en particulier les dérivés de xylose.

On pourra également utiliser avantageusement un émulsionnant à base d'alkylpolyglycosides et de diols gras, comprenant notamment :

- 5 à 95 parties en poids d'un mélange d'alkylpolyglycosides constitué des produits de réaction d'un saccharide et d'un dimerdiol ayant 36 atomes de carbone ;
- 95 à 5 parties en poids d'un dimerdiol ayant 36 atomes de carbone.

Les émulsionnants préférés, répondant à la définition ci-dessus comprennent :

- 5 à 60 parties en poids du mélange précité d'alkylpolyglycosides ; et
- 95 à 40 parties en poids de dimerdiol ayant 36 atomes de carbone.

Le mélange d'alkylpolyglycosides constitué des produits de réaction d'un saccharide et d'un dimerdiol ayant 36 atomes de carbone est en fait constitué d'un mélange en toutes proportions d'hydroxyalkylpolyglycosides (produits résultant de l'acétalisation de l'un des deux groupes hydroxyles du dimerdiol) et de polyglycosylalkylpolyglycosides (produits résultant de l'acétalisation des deux groupes hydroxyles du dimerdiol).

Ces alkylpolyglycosides peuvent être représentés, respectivement par les formules I et II suivantes :

$$HO\text{-}R\text{-}O(G)_n \qquad (I)$$

$$(G)_m\text{-}OR\text{-}O\text{-}(G)_p \qquad (II)$$

dans lesquelles :

G représente un reste de saccharide ;
R représente un groupe disubstitué dérivé de l'alcool dimère provenant de l'hydrogénation de l'acide dimère ;
n, m et p représentent le degré de polymérisation moyen de chaque reste de saccharide.

Le produit connu sous la dénomination "acide dimère" est un acide dibasique ayant 36 atomes de carbone dont le composé majoritaire peut être représenté par la formule :

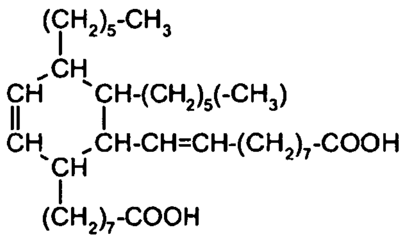

Les alkylpolyglycosides précités peuvent comporter, à titre de reste de saccharide, un reste de glucose ou dextrose, fructose, galactose, mannose, ribose, xylose, de préférence un reste de glucose ou de xylose.

Il est en outre à noter que chaque unité de la partie polyoside des alkylpolyglycosides précités peut être sous forme anomérique $\alpha$ ou $\beta$, et le reste de saccharide peut être de type furanoside ou pyranoside.

Le degré de polymérisation moyen de chaque reste de saccharide est généralement compris entre 1,05 et 2,5, de préférence encore entre 1,1 et 2.

L'expression "alkylpolyglycoside" utilisée dans le cadre de la présente demande désigne donc indifféremment un alkylmonooside (degré de polymérisation égal à 1) ou un alkylpolyglycoside (degré de polymérisation supérieur à 1).

Le dimerdiol utilisé pour la préparation du tensioactif émulsionnant ci-dessus est un diol provenant de l'hydrogénation de l'acide dimère.

Il est notamment commercialisé par la Société COGNIS sous la dénomination SPEZIOL® C 36/2.

Ce composé, en raison de son origine, peut contenir des proportions mineures d'impuretés. De telles impuretés peuvent être présentes en des quantités allant jusqu'à 30 % en poids du poids total de diol.

Par conséquent, les tensioactifs émulsionnants à base d'alkylpolyglycosides et de diols gras peuvent comprendre, en des proportions mineures correspondantes, de telles impuretés, ou les produits de réaction de ces impuretés avec un saccharide.

Les tensioactifs émulsionnants à base d'alkylpolyglycosides et de diols gras utilisables dans le cadre de la présente invention peuvent être préparées par simple mélange de leurs constituants en des proportions prédéterminées souhaitées.

A l'échelle industrielle, on les préparera de préférence selon l'une des deux voies classiquement utilisées pour

la synthèse des alkylpolyglycosides, et par exemple par réaction, en milieu acide, entre le dimerdiol et un saccharide disposant d'un OH anomérique, tel que le glucose ou le dextrose.

Le cas échéant, cette synthèse pourra être complétée par des opérations de neutralisation, de filtration, de distillation ou d'extraction partielle du diol gras en excès ou de décoloration.

Il pourra également être particulièrement avantageux d'utiliser un tensioactif émulsionnant à base d'alkylpolyxyloside, tel que décrit dans la demande EP-A-1142901, de formule :

$$R\text{-}O\text{-}(X)_p$$

dans laquelle :

p représente un nombre décimal compris entre 1 et 5,
X représente le reste du xylose, et
R représente un radical alkyl ramifié :

$$CH(C_nH_{2n+1})(C_mH_{2m+1})\text{-}CH_2\text{-}$$

dans lequel m est un nombre entier compris entre 6 et 18, n est un nombre entier compris entre 4 et 18 et la somme n + m est supérieure ou égale à 14 ;

ou bien, dans un mode de réalisation particulièrement préférentiel, une composition consistant en un mélange d'au moins deux composés tels que définis ci-dessus ;

ou bien encore une composition comprenant

plus de 0% en poids et moins de 100% en poids, de préférence de 1% à 60% en poids, d'un composé ou d'un mélange de composés définis ci-dessus et

plus de 0% en poids et moins de 100% en poids, de préférence de 40% à 99% en poids, d'un composé ou d'un mélange de composés de formule ROH dans laquelle R a la signification mentionnée précédemment.

De manière particulièrement préférentielle, on utilise un mélange d'alkylpolyxyloside(s) $R\text{-}O\text{-}(X)_p$ et de son (leurs) alcool(s) correspondant(s) ROH, dans les proportions indiquées ci-dessus.

Selon un aspect préféré de l'invention, on utilise un système émulsionnnant contenant au moins un tensioactif émulsionnant choisi parmi les alkylpolyglycosides, les compositions d'alkylpolyglycoside(s) et d'alcool(s) gras, les esters de polyols (tels que les polyglycols et polyglycérols et éventuellement d'autres polyols) éventuellement alcoxylés tels que les polyhydroxystéarates de polyols (tels que les polyglycols ou les polyglycérols) éventuellement alcoxylés, les copolymères polyéthylèneglycol-alkylglycols.

De manière encore plus préférée, on utilise un système émulsionnant contenant un ester de polyglycérol (tel qu'un polyhydroxystéarate de polyglycérol) éventuellement alcoxylé, un polyhydroxystéarate de polyglycol éventuellement alcoxylé, ou un copolymère polyéthylèneglycol-alkylglycol, en combinaison avec un alkylpolyglycoside ou une composition d'alkylpolyglycoside(s) et d'alcool(s) gras.

L'émulsion eau-dans-huile comprend généralement jusqu'à 10 % en poids, de préférence jusqu'à 5% en poids, et encore plus préférentiellement de 0,5 à 5% en poids du système émulsionnant comprenant un ou plusieurs tensioactifs émulsionnants.

Indépendamment de la phase grasse, on prépare une phase aqueuse gélifiée contenant un polymère de type polyélectrolyte dont les sites ioniques sont associés à leurs contre-ions. Parmi les polymères de type polyélectrolyte susceptibles d'être utilisés dans le cadre de la présente invention, on peut citer:

- des homopolymères à base d'un monomère possédant une fonction acide fort, partiellement ou totalement salifiée,
- des homopolymères à base d'un monomère possédant une fonction acide faible, partiellement ou totalement salifiée,
- des homopolymères à base d'un monomère cationique,
- des copolymères à base d'au moins un monomère possédant une fonction acide fort partiellement ou totalement salifiée, copolymérisé :
  - o soit avec au moins un monomère possédant une fonction acide faible partiellement ou totalement salifiée,
  - o soit avec au moins un monomère neutre,
- des copolymères à base d'un monomère cationique copolymérisé avec au moins un monomère neutre,
- des copolymères à base d'au moins un monomère possédant une fonction acide faible partiellement ou totalement salifiée copolymérisé :

ο soit avec au moins un monomère possédant une fonction acide faible partiellement ou totalement salifiée,
ο soit avec au moins un monomère neutre.

Dans ce contexte, la phrase "partiellement ou totalement salifiée" signifie que les fonctions acide fort ou acide faible sont partiellement ou totalement salifiées sous forme notamment de sel de métal alcalin, tel que le sel de sodium ou le sel de potassium, de sel d'ammonium ou de sel d'amino-alcool, tel que par exemple, le sel de monoéthanolamine.
La fonction acide fort du monomère peut notamment être la fonction acide sulfonique ou la fonction acide phosphonique, lesdites fonctions étant partiellement ou totalement salifiées.
Ledit monomère sera avantageusement choisi parmi l'acide styrène sulfonique ou le méthacrylate de 2-sulfoéthyle, l'acide styrène phosphonique, partiellement ou totalement salifié, l'acide-2-méthyl-[(1-oxo-2-propényl)amino] 1-propane sulfonique (AMPS) partiellement ou totalement salifié sous forme de sel de sodium, de sel d'ammonium ou de sel de monoéthanolamine.
La fonction acide faible du monomère peut notamment être la fonction acide carboxylique partiellement ou totalement salifiée. Ledit monomère peut notamment être choisi parmi l'acide acrylique, l'acide méthacrylique, l'acide itaconique ou l'acide maléique partiellement ou totalement salifié sous forme de sel de sodium, de sel de potassium, de sel d'ammonium ou de sel de monoéthanolamine.
Lorsque le polymère est un copolymère à base d'un monomère possédant une fonction acide fort partiellement ou totalement salifiée, copolymérisé, avec au moins un monomère neutre, ledit monomère neutre est notamment choisi parmi l'acrylamide, le méthacrylamide, la vinylpyrrolidone, l'acrylate de (2-hydroxyéthyle), l'acrylate de (2,3-dihydroxypropyle), le méthacrylate de (2-hydroxyéthyle) ou le méthacrylate de (2,3-dihydroxypropyle) ou un dérivé éthoxylé de poids moléculaire compris entre 400 et 1000, de chacun de ces esters hydroxylés, le tris(hydroxyméthyl)-acrylamidométhane ou le tris(hydroxyméthyl) méthacrylamidométhane ou un dérivé éthoxylé de poids moléculaire compris entre 400 et 1500, de chacun de ces amides.
Le fait que la phase aqueuse des émulsions selon la présente invention comprend un polyélectrolyte dont les sites ioniques sont associés à leurs contre-ions contribue à ce que l'apport complémentaire de sels minéraux ne soit pas nécessaire.
Les polymères cités ci-dessus peuvent être "ramifiés" ou "réticulés". Par "polymère ramifié", on désigne un polymère non linéaire qui possède des chaînes pendantes de manière à obtenir, lorsque ce polymère est mis en solution dans l'eau, un fort état d'enchevêtrement conduisant à des viscosités à bas gradient très importantes. Par "polymère réticulé", on désigne un polymère non linéaire se présentant à l'état de réseau tridimensionnel insoluble dans l'eau, mais gonflable à l'eau et conduisant donc à l'obtention d'un gel chimique.
Lorsque le polymère est réticulé et/ou ramifié, l'agent de réticulation et/ou l'agent de ramification est notamment choisi parmi les composés diéthyléniques, polyéthyléniques, et tout particulièrement parmi l'acide diallyloxyacétique ou un de ses sels et notamment son sel de sodium, la triallylamine, le triméthylol propanetriacrylate, le diméthacrylate d'éthylèneglycol, le diacrylate de diéthylène glycol, la diallylurée ou le méthylène bis(acrylamide).
L'agent de réticulation et/ou de ramification est généralement utilisé dans la proportion molaire exprimée par rapport aux monomères mis en oeuvre, de 0,005% à 1% , notamment de 0,01% à 0,2% et plus particulièrement de 0,01% à 0,1%.
De façon générale les polymères de type polyélectrolytes qui conviennent pour la réalisation d'émulsions selon la présente invention sont donc des copolymères ou homopolymères, qui peuvent ou non être réticulés ou ramifiés, comprenant des monomères possédant une fonction acide fort ou acide faible partiellement ou totalement salifiée, ou une fonction cationique.
Parmi les polymères de type polyélectrolytes qui conviennent tout particulièrement pour la mise en oeuvre du procédé de la présente invention, nous pouvons citer les dérivés de l'acrylamide, de l'acide acrylique et de la vinylpyrrolidone tels que les copolymères de l'acide acrylique et de l'acide-2-méthyl-[(1-oxo-2-propényl)amino] 1-propane sulfonique (AMPS), les copolymères de l'acrylamide et de l'acide-2-méthyl-[(1-oxo-2-propényl)amino] 1-propane sulfonique, les copolymères de l'acide-2-méthyl-[(1-oxo-2-propényl)amino] 1-propane sulfonique et de l'acrylate de (2-hydroxyéthyle), l'homopolymère de l'acide-2-méthyl-[(1-oxo-2-propényl)amino] 1-propane sulfonique, l'homopolymère de l'acide acrylique, les copolymères du chlorure d'acryloyl éthyl triméthyl ammonium et de l'acrylamide, les copolymères de l'AMPS et de la vinylpyrrolidone, les copolymères de l'acide acrylique et d'acrylates d'alkyle dont la chaîne carbonée comprend entre dix et trente atomes de carbone, les copolymères de l'AMPS et d'acrylates d'alkyle dont la chaîne carbonée comprend entre dix et trente atomes de carbone.
De tels polymères sont généralement préparés par un processus de polymérisation en phase inverse, et sont notamment commercialisés respectivement sous les dénominations SIMULGEL® EG, SEPIGEL® 305, SIMULGEL® NS, SIMULGEL® 800 et SIMULGEL® A par la société SEPPIC. Ils impliquent des formes partiellement ou totalement salifiées des monomères acides. Le ou les monomères correspondant(s) est (sont) dissous dans des gouttes d'eau dispersées dans une phase grasse à l'aide d'un tensioactif. Dans ce type de polymérisation, chaque goutte d'eau dans l'émulsion eau dans huile constitue en soi un "petit réacteur". Ce système minimise les probabilités de réactions de

terminaison et conduit à des chaînes polymériques de plus haut poids moléculaire. A la fin de la réaction on ajoute un tensioactif hydrophile et de l'eau. Le polymère se déploie, n'étant plus contraint par la taille de la goutte d'eau dans laquelle il a été synthétisé, conduisant à un "solide mou", se caractérisant par une structure tridimensionnelle.

Ce procédé de préparation fait appel à des tensioactifs, tels que des esters de sorbitan, des esters de mannitan, des polyhydroxystéarates de polyglycols, de polyglycérols ou de polyols, des alkanolamides sur chaînes grasses linéaires ou ramifiées, des esters de sorbitan éthoxylés, des esters de mannitan éthoxylés, des nonylphénols éthoxylés, des octylphénols éthoxylés. De ce fait, les tensioactifs cités précédemment peuvent être présents dans la phase aqueuse gélifiée lors de la mise en oeuvre du procédé de l'invention.

Avantageusement le poids sec dudit polyélectrolyte constitue entre 0,1% et 4% et de préférence entre 0,5 % et 2% du poids de la phase aqueuse, si le procédé de préparation d'un tel polyélectrolyte résulte d'un processus de polymérisation précipitante ; ou le poids sec dudit polyélectrolyte constitue entre 0,25 % et 4% et de préférence entre 0,5% et 2% du poids de la phase aqueuse si le procédé de préparation d'un tel polyélectrolyte résulte d'un processus de préparation de polymérisation en émulsion inverse.

Les polymères de type polyélectrolyte utilisés dans le procédé de la présente invention ont un comportement rhéofluidifiant (non-Newtonien), c'est-à-dire que la viscosité observée varie en fonction du gradient de cisaillement.

Sans vouloir être lié par une théorie particulière, on pense que ce comportement rhéofluidifiant (non-Newtonien) est vraisemblablement lié à la structure tridimensionnelle des polymères de type polyélectrolyte, qui influe également sur les propriétés physiques des émulsions. Il existe donc une corrélation entre le comportement des polymères en matière de viscosité et leur capacité à faciliter la formation d'émulsions.

Avantageusement, les polymères de type polyélectrolyte utilisables dans le cadre de la présente invention présentent un comportement rhéologique non-Newtonien en solution, caractérisé par un indice de gradient compris entre 0,1 et 0,7, et préférentiellement entre 0,2 et 0,5.

Avantageusement, la phase aqueuse gélifiée obtenue par mise en solution du polymère de type polyélectrolyte présentera une viscosité comprise entre 0,5 et 300 Pa.s, préférentiellement entre 1,0 et 150 Pa.s et plus particulièrement entre 5 et 100 Pa.s, mesurée sur un viscosimètre BROOKFIELD LV (6 rpm, 20°C).

L'émulsion eau-dans-huile conforme à la présente invention peut également contenir, de façon optionnelle, jusqu'à 10 % en poids d'un stabilisant.

Parmi les agents stabilisants susceptibles d'être utilisés dans le cadre de la présente invention, on peut citer l'huile de ricin hydrogénée; l'acide stéarique et ses sels métalliques comme le stéarate d'aluminium ; les silices hydrophobes ; les polymères tels que les produits commercialisés sous la dénomination KRATON® POLYMERS par la société KRATON ; les argiles comme l'hectorite ou la bentonite ; les amidons modifiés hydrophobes comme par exemple le produit commercialisé sous la dénomination DRY FLO PC® par la société NATIONAL STARCH ; les polyméthylméthacrylates réticulés ou non comme les MICROPEARL commercialisés par la société SEPPIC, les polyamides comme l'Orgasol 2002 commercialisé par la société ATOCHEM.

Des cires d'origine végétale, animale or minérale, telles que la cire d'abeille, la cire de carnauba, la cire de candellila ou l'ozokérite, sont également susceptibles d'être utilisées dans le cadre de la présente invention, même si pour des raisons pratiques, elles ne représentent pas un aspect préféré de l'invention.

D'une façon connue en soi, ces émulsions peuvent en outre comprendre un ou plusieurs composés choisis parmi les humectants, comme par exemple la glycérine, les glycols, le sel sodique de l'acide 5-carboxylique de la 2-pyrrolidone, les conservateurs comme par exemple les produits connus sous la dénomination SEPICIDE® et commercialisés par la société SEPPIC, les colorants, les parfums, les actifs cosmétiques comme par exemple les vitamines et les dérivés de vitamines hydrosolubles, les vitamines et les dérivés de vitamines liposolubles, des oligosaccharides, des protéines, des polypeptides, des acides aminés, des dérivés N-acylés d'acides aminés et/ou de polypeptides et/ou de protéines, des extraits de plantes, des extraits d'algues marines, les filtres solaires minéraux ou organiques, les charges minérales comme le mica, la silice et le talc, les charges synthétiques comme les nylons et les poly(méthacrylate de méthyle) réticulés ou non, les élastomères silicone, les séricites.

L'émulsion eau-dans-huile peut également comprendre un ou plusieurs sels minéraux électrolytes, comme par exemple le chlorure de magnésium, le sulfate de magnésium, le borate de sodium ou le chlorure de sodium, en une quantité allant de 0,1% à 5% en poids. Ces électrolytes ne sont toutefois pas indispensables à l'obtention des émulsions conformes à l'invention.

Ces composés pourront être introduits dans la phase aqueuse ou dans la phase huileuse, selon leur affinité pour ces phases.

Les émulsions selon l'invention sont obtenues en ajoutant la phase grasse sur la phase aqueuse. Il est possible d'opérer à des températures allant jusqu'à 80°C. Cependant il est avantageux de minimiser la dépense d'énergie que représente le chauffage des phases et on préférera donc réaliser l'émulsion à une température inférieure à 55°C. Encore plus préférentiellement, on ajoutera la phase grasse sur la phase aqueuse gélifiée à une température comprise entre 20°C et 35°C, c'est-à-dire à température ambiante.

L'émulsion selon l'invention peut être réalisée en mélangeant les deux phases à une vitesse d'agitation dépassant 1500 tours par minute. Il est avantageux cependant de minimiser l'énergie dépensée dans l'agitation utilisée pour réaliser l'émulsion. On préférera donc une vitesse d'agitation inférieure à 1000 tours par minute, et on préférera tout particulièrement une vitesse d'agitation comprise entre 80 et 800 tours par minute. Encore plus préférentiellement, on choisira une vitesse d'agitation comprise entre 80 et 450 tours par minute, tout particulièrement entre 80 et 330 tours par minute.

Les émulsions conformes à l'invention, qui sont stables dans le temps, présentent avantageusement un indice de polydispersité supérieur à 41%, de préférence supérieur à 45% et plus préférentiellement supérieure ou égale à 51%.

Dans ce contexte, la polydispersité est déterminée par analyse granulométrique sur une forme diluée de l'émulsion à l'aide d'un granulomètre laser de type MALVERN MASTERSIZER.

En particulier, par la mise en oeuvre des conditions opératoires du procédé de la présente invention, il est possible:

- de combiner les phases grasse et aqueuse qui sont toutes les deux à température ambiante,
- d'effectuer cette combinaison de phases sans contrainte quant au débit d'introduction d'une phase dans l'autre,
- de réaliser l'émulsion avec une agitation relative douce au moyen de systèmes d'agitation de géométrie simple (ancre, hélice marine, défloculeuse de type Rayneri),
- d'éviter l'utilisation de substances supplémentaires souvent indispensables pour la préparation d'émulsions E/H à forte teneur en phase aqueuse de l'art antérieur, comme notamment les cires microcristallines et les sels minéraux électrolytes.

De plus, dans le procédé selon l'invention l'ordre d'ajout des phases est inversé par rapport à l'ordre habituel de préparation d'une émulsion de type eau dans huile (E/H). Le fait d'ajouter la phase grasse sur la phase aqueuse représente un gain de productivité en lui-même. En effet, la phase grasse est d'un volume moindre que la phase aqueuse, ce qui peut permettre l'utilisation d'une seule cuve de préparation, et présente une viscosité bien inférieure.

L'émulsion E/H conforme à l'invention peut être avantageusement utilisée dans une préparation cosmétique, dermocosmétique, pharmaceutique ou vétérinaire. L'émulsion E/H selon l'invention peut également être utilisée dans une préparation détergente.

Selon un aspect particulier, l'émulsion E/H selon l'invention est une émulsion solaire, dans laquelle un ou plusieurs filtres solaires (des substances permettant d'empêcher les rayonnement ultra-violets d'atteindre la peau de l'utilisateur) est (sont) incorporé(s) dans la phase grasse et/ou dans la phase aqueuse gélifiée.

Les émulsions solaires doivent satisfaire à un certain nombre de critères :

- elles doivent présenter un effet photoprotecteur suffisant. On quantifie ce facteur par la mesure de la capacité d'une composition solaire à réduire l'érythème provoqué par les rayonnements ultra-violets ;
- il est souhaitable qu'elles possèdent des propriétés de résistance à l'eau, c'est-à-dire qu'une fois étalées sur la peau, elles conservent le plus possible leur fonction photoprotectrice après la baignade ;
- pour le confort de l'utilisateur, il est également souhaitable que les compositions topiques solaires possèdent des qualités pratiques et sensorielles. Elles doivent notamment être faciles à étaler, ne doivent pas provoquer une sensation de gras, ni être collantes.

Un grand nombre de compositions solaires a été développé à base d'émulsions de type huile-dans-eau. Avec suffisamment de phase grasse, ce type d'émulsion a l'avantage de faciliter la dissolution de filtres solaires, qui sont pour la plupart des composés organiques lipophiles ou lipodispersibles, et permet également d'atteindre de bonnes propriétés sensorielles : toucher doux et étalement facile.

La résistance à l'eau des émulsions huile-dans-eau est cependant faible. Il peut par conséquent s'avérer nécessaire d'ajouter des additifs qui "structurent" ou "rigidifient" la phase aqueuse, tels que la poly(vinylpyrrolidone), les polyacrylates, les polyacrylamides ou encore les huiles siliconées qui augmentent les propriétés hydrofuges du film obtenu en étalant l'émulsion.

L'ajout de tels additifs est souvent nuisible en ce qui concerne les propriétés sensorielles recherchées.

Il a donc été envisagé de formuler des émulsions de type eau-dans-huile. De telles émulsions présentent toutefois l'inconvénient d'être difficiles à étaler et sont perçues comme grasses au niveau sensoriel.

Les présents inventeurs ont découvert que l'utilisation d'émulsions de type eau-dans-huile selon la présente invention permet de répondre de façon satisfaisante à l'ensemble des critères mentionnés ci-dessus.

Il est notamment possible de combiner un niveau de photoprotection élevé avec une résistance à l'eau importante tout en gardant une facilité d'étalement acceptable et en respectant d'autres paramètres sensoriels (toucher non gras, non collant).

Il est possible d'utiliser n'importe laquelle des huiles précédemment évoquées pour la préparation d'émulsions solaires selon la présente invention. On préfère cependant pour cette application utiliser une ou plusieurs huiles synthétiques, choisies parmi le groupe constitué des esters d'acides gras tels que le néopentanoate d'isodécyle, le myristate de butyle, le myristate de propyle, le myristate de cétyle, le palmitate d'isopropyle, le palmitate de 2-éthylhexyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les monoglycérides, diglycérides et triglycérides d'acides gras comme le triheptanoate de glycérol et le caprylic capric triglycéride, le benzoate d'alkyle en $C_{12}$-$C_{15}$, l'adipate de diisopropyle, le cocoate d'éthylhexyle, l'adipate de diisopropyle ou d'éthylhexyle, le sébacate de diisopropyle ou de diéthylhexyle, le lactate de lauryle ou de myristyle, le maléate de diéthylhexyle.

Les émulsions solaires selon la présente invention comprennent un système photoprotecteur comprenant un ou plusieurs filtres solaires, qui peuvent être incorporés dans la phase grasse et/ou dans la phase aqueuse gélifiée. Généralement les émulsions solaires comprennent environ 2% à environ 40% en poids, de préférence environ 5% à environ 20% en poids de filtre(s) solaire(s).

Lorsque la phase aqueuse gélifiée de l'émulsion comprend un (des) filtre(s) solaire(s), celui-ci (ceux-ci) est (sont) généralement directement dispersé(s) dans la phase aqueuse gélifiée s'il(s) est (sont) liquide(s) ou, s'il(s) est (sont) solide(s), il(s) est (sont) généralement préalablement dispersé(s) dans un solvant tel que l'éthanol ou dans un autre filtre solaire liquide qui le(s) solubilise avant d'être incorporé(s) dans le gel aqueux.

Les filtres solaires peuvent être de nature organique ou de nature inorganique, et il est possible de combiner filtres organiques et inorganiques dans une même émulsion solaire.

Parmi les filtres organiques, on peut distinguer la famille des dérivés de l'acide benzoïque comme les acides para-aminobenzoïques (PABA), notamment les esters de monoglycérol de PABA, les esters éthyliques de N,N-propoxy PABA, les esters éthyliques de N,N-diéthoxy PABA, les esters éthyliques de N,N-diméthyl PABA, les esters méthyliques de N,N-diméthyl PABA, les esters butyliques de N,N-diméthyl PABA; la famille des dérivés de l'acide anthranilique comme l'homomenthyl-N-acétyl anthranilate ; la famille des dérivés de l'acide salicylique comme le salicylate d'amyle, le salicylate d'homomenthyle, le salicylate d'éthylhexyle, le salicylate de phényle, le salicylate de benzyle, le salicylate de p-isopropanolphényle ; la famille des dérivés de l'acide cinnamique comme le cinnamate d'éthylhexyle, le cinnamate d'éthyl-4-isopropyle, le cinnamate de méthyl-2,5-diisopropyle, le cinnamate de p-méthoxypropyle, le cinnamate de p-méthoxyisopropyle, le cinnamate de p-méthoxyisoamyle, le cinnamate de p-méthoxyoctyle (le cinnamate de p-méthoxy 2-éthylhexyle), le cinnamate de p-méthoxy 2-éthoxyéthyle, le cinnamate de p-méthoxycyclohexyle, le cinnamate d'éthyl-$\alpha$-cyano-$\beta$-phényle, le cinnamate de 2-éthylhexyl- $\alpha$-cyano-$\beta$-phényle, le cinnamate de diparaméthoxy mono-2-éthylhexanoyl de glycéryle ; la famille des dérivés de la benzophénone comme la 2,4-dihydroxybenzophénone, la 2,2'-dihydroxy-4-méthoxybenzophénone, la 2,2',4,4'-tétrahydroxybenzophénone, la 2-hydroxy-4-méthoxybenzophénone, la 2-hydroxy-4-méthoxy-4'-méthylbenzophénone, la 2-hydroxy-4-méthoxybenzophénone-5-sulfonate, la 4-phénylbenzophénone, le 2-éthylhexyl-4'-phénylbenzophénone-2-carboxylate, la 2-hydroxy-4-n-octyloxybenzophénone, la 4-hydroxy-3-carboxybenzophénone ; le 3-(4'-méthylbenzylidène)-d,l-camphre, le 3-(benzylidène)-d,l-camphre, le benzalkonium méthosulfate camphre ; l'acide urocanique, l'urocanate d'éthyle ; la famille des dérivés de l'acide sulfonique comme l'acide sulfonique 2-phénylbenzimidazole-5 et ses sels ; la famille des dérivés de la triazine comme l'hydroxyphényl triazine, l'éthylhexyloxyhydroxyphényl-4-méthoxyphényltriazine, le 2,4,6-trianillino-(p-carbo-2'-éthylhexyl-1'-oxy)-1,3,5-triazine, le 4,4-((6-(((1,1-diméthyléthyl) amino)carbonyl)phenyl)amino)-1,3,5-triazine-2,4-diyl diimino) bis-(2-éthylhexyl) ester de l'acide benzoïque, le 2-phényl-5-méthylbenzoxazole, le 2,2'-hydroxy-5-méthylphénylbenzotriazole, le 2-(2'-hydroxy-5'-t-octylphényl)benzotriazole, le 2-(2'-hydroxy-5'-méthyphényl)benzotriazole; la dibenzazine; le dianisoylméthane, le 4-méthoxy-4"-t-butylbenzoylméthane ; la 5-(3,3-diméthyl-2-norbornylidène)-3-pentan-2-one ; la famille des dérivés du diphénylacrylate comme le 2-éthylhexyl-2-cyano-3,3-diphényl-2-propènoate, l'éthyl-2-cyano-3,3-diphényl-2-propènoate ; la famille des polysiloxanes comme le malonate de benzylidène siloxane.

Parmi les filtres inorganiques, également appelés "écrans minéraux", on peut distinguer, les oxydes de titane, les oxydes de zinc, l'oxyde de cérium, l'oxyde de zirconium, les oxydes de fer jaune, rouge ou noir, les oxydes de chrome. Ces écrans minéraux peuvent être micronisés ou non, avoir subi ou non des traitements de surface et être éventuellement présentés sous formes de pré-dispersions aqueuses ou huileuses.

De manière avantageuse, des émulsions solaires selon la présente invention comprennent une ou plusieurs charges minérales dans la phase huileuse.

L'invention sera illustrée par les exemples suivants.

<u>**EXEMPLE 1 : Préparation d'une émulsion E/H**</u>

On prépare séparément deux phases ayant les compositions suivantes :

| | |
|---|---|
| Phase grasse | |
| Alkyl polyxylosides sur Isofol® 20 préparé selon EP1142901 | 1,6 % |

(suite)

| | |
|---|---|
| Phase grasse | |
| PEG45 dodecylglycol copolymère (ELFACOS® ST9) | 0,4 % |
| Triglycéride C8-C10 | 8,0 % |
| Phase aqueuse | |
| Eau | qs 100 % |
| Glycérine | 5 % |
| SIMULGEL® EG | 2 % |

La phase grasse est chauffée modérément (50°C max.) jusqu'à limpidité du mélange des trois constituants. Cette phase grasse peut être stockée à température ambiante pendant plusieurs jours sans provoquer de cristallisation des différents tensioactifs présents.

Le gel aqueux comprenant l'eau, un polymère hydrosoluble présenté en émulsion inverse sous l'appellation commerciale Simulgel® EG, et la glycérine est préparé à température ambiante sous agitation conventionnelle pour ce type de préparation (défloculeuse RAYNERI), à une température de 18-25°C, avec une vitesse d'agitation de 300 tours/min.

La phase grasse est ajoutée en une seule fois sur le gel, à température ambiante et à une vitesse d'agitation modérée (200 à 300 tours/min.) avec un agitateur équipé d'un mobile de type ancre. Cette agitation est alors maintenue pendant dix minutes et aucune étape de refroidissement n'est nécessaire.

Par ailleurs l'émulsion obtenue est une émulsion eau dans huile qui présente du fait de sa forte teneur en eau, un fort caractère rhéofluidifiant associé à un toucher non collant et frais.

**<u>EXEMPLE 2 : Préparation d'une émulsion E/H</u>**

Le même mode opératoire que celui décrit dans l'exemple 1 est utilisé dans un réacteur pilote de 8 kg, équipé d'un mélangeur planétaire. La vitesse d'émulsification avec un tel système a été de 100 tours/min et la phase de maintien, après la fin de l'ajout de la phase grasse, a duré trente minutes.

**<u>EXEMPLE 3: Influence de l'énergie apportée par la qualité de l'agitation</u>**

Les conditions de l'exemple 1 ont été reproduites en utilisant un agitateur de type rotor stator, fonctionnant à une vitesse de 4000 tours/minutes, pour réaliser l'émulsion.

**<u>EXEMPLE 4: Influences combinées de la qualité de l'agitation et de la température d'émulsification</u>**

La phase grasse est chauffée à 80°C jusqu'à limpidité du mélange des trois constituants.

Le gel aqueux comprenant l'eau, un polymère hydrosoluble présenté en émulsion inverse sous l'appellation commerciale Simulgel® EG, et la glycérine est préparé à température ambiante sous agitation conventionnelle pour ce type de préparation (défloculeuse RAYNERI). Il est ensuite chauffé à 80°C.

La phase grasse est ajoutée en une seule fois sur le gel, et les conditions d'agitation de l'exemple 3 ont été reproduites.

L'émulsion est ensuite refroidie sous agitation modérée avec une ancre pendant 20 minutes.
Les propriétés des émulsions obtenues selon les exemples 1 à 4 sont rassemblées dans le Tableau 1.

Tableau 1

| | Exemple 1 | Exemple 2 | Exemple 3 | Exemple 4 |
|---|---|---|---|---|
| Quantité fabriquée | 200g | 8kg | 200g | 200g |
| Température d'émulsification | 20-25°C | 20-25°C | 20-25°C | 80°C |
| Vitesse d'agitation lors de l'émulsification | 300 tr/min | 100 tr/min | 4 000 tr/min | 4 000 tr/min |
| Caractéristiques de l'émulsion obtenue/ SENS (1) | E/H | E/H | E/H | E/H |
| VISCOSITE (2) | 55,6 Pa.s | 58,0 Pa.s | 51,2 Pa.s | 51,2 Pa.s |
| (1) méthode: 1g d'émulsion est dilué dans 5g d'eau déminéralisée. Si l'émulsion est non miscible à l'eau, elle est E/H .<br>(2) méthode : BROOKFIELD LV 6rpm (à 20°C) | | | | |

La comparaison des exemples 3 et 4 avec l'exemple 1 montre que le sens de l'émulsion E/H et l'ordre de grandeur de la viscosité de l'émulsion obtenue sont similaires quelle que soit la quantité d'énergie fournie au système.
D'autre part, la comparaison des exemples 1 et 2 montre que l'extrapolation du procédé à une échelle "pilote" se traduit par des performances similaires, sans modification significative des conditions opératoires relatives à la phase d'émulsification.

**EXEMPLE 5: Etude du comportement rhéologique d'un gel aqueux utilisable dans le cadre de la présente invention**

Le comportement rhéologique de différentes phases aqueuses gélifiées, contenant un polymère de type polyélectrolyte, a été étudié en l'absence d'une phase organique à l'aide d'un rhéomètre à contrainte imposée CSL500, commercialisé par la société TA Instruments. Les mesures de viscosité sont réalisées à 20°C. Les indices de gradient, indiqués dans le tableau 2, sont obtenus par calcul de convexité de la courbe analysée selon le modèle mathématique de la loi de puissance.

Tableau 2

| POLYELECTROLYTE ETUDIE | QUANTITE EN POIDS SEC DANS LA PHASE AQUEUSE (%) | Indice de gradient | Viscosité Brookfield LV 6 RPM (Pa.s) |
|---|---|---|---|
| Copolymère de l'AMPS et de l'acide acrylique | 0,96 | 0,28 | 70 |
| Copolymère de l'AMPS et de l'acrylamide | 0,8 | 0,32 | 60 |
| Copolymère de l'AMPS et de l'HydroxyEthyl Acrylate | 1,06 | 0,31 | 73 |
| Homopolymère de l'AMPS | 0,95 | 0,34 | 61 |
| Copolymère de l'AMPS et de la vinyl pyrolidone | 0,9 | 0,34 | 70 |
| Copolymère de l'acide acrylique et de C10-30 alkylacrylates | 0,7 | 0,4 | 69 |
| Homopolymère de l'acide acrylique | 1,5 | 0,48 | 61 |

Ces résultats montrent que les gels aqueux, obtenus à partir de polymères de type polyélectrolyte, présentent un caractère rhéofluidifiant. Ce profil est illustré par la mesure de l'indice de gradient
On observe que l'indice de gradient est généralement inférieur à 0,5 pour les gels à base de polymères polyélectrolytes. D'autres études, dont les résultats ne sont pas indiqués, montrent que les gels aqueux obtenus à partir de polyéthylèneglycols, présentent un indice de gradient proche de 1, traduisant ainsi un caractère quasi newtonien.

**EXEMPLE 6: Etude de la polydispersité d'une émulsion selon la présente invention**

On prépare séparément deux phases ayant les compositions suivantes :

Phase grasse

- FLUIDANOV® 20X[1] 0,50%
  (octyldodécanol polyxyloside et octyldodécanol)
- ARLACEL® P 135 0,10%
  (polyhydroxystéarate de PEG 1500)
- LANOL® 99 4,40%
  (isononanoate d'isononyle)

Phase aqueuse

- SIMULGEL® EG 2,85%
  (sodium acrylate / sodium acryloyl dimethyl taurate / isohexadecane / Polysorbate 80)
- Eau 92,15%
  [1] Préparé selon EP-A-1142901

Ces deux phases ont été combinées en suivant le mode opératoire de l'exemple 1 ci-dessus.

La polydispersité de l'émulsion eau-dans-huile obtenue a été mesurée selon la méthode indiqué précédemment (analyse granulométrique sur une forme diluée de l'émulsion à l'aide d'un granulomètre laser de type MALVERN MASTERSIZER).

L'indice de polydispersité de l'émulsion était supérieur à 51%. Par ailleurs, l'indice de gradient de la phase aqueuse de cette émulsion eau-dans-huile était de 0,28.

**<u>EXEMPLE 7: Influence de la viscosité du gel aqueux sur le sens de l'émulsion</u>**

On prépare séparément deux phases ayant les compositions suivantes :

| | |
|---|---|
| Phase grasse | |
| Alkyl polyxylosides sur Isofol® 20 préparé selon EP1142901 | 2,4 % |
| PEG45 dodécylglycol copolymère (ELFACOS® ST9) | 0,6 % |
| Triglycéride C8-C10 | 12,0 % |
| Phase aqueuse | |
| Eau | qs 100 % |
| SIMULGEL® EG | x % |

Les conditions de l'exemple 1 ont été reproduites en utilisant un agitateur de type ancre, fonctionnant à une vitesse de 300 tours/minute, pour réaliser l'émulsion.

La viscosité de la phase aqueuse et le sens résultant de l'émulsion, préparée selon le mode opératoire décrit ci-dessus, sont consignés dans le tableau 3.

Tableau 3

| Quantité de SIMULGEL® EG Employée (1) | Quantité de polyélectrolyte Employée (1) | Viscosité de la phase aqueuse Pa.s | Caractéristique de l'émulsion/Sens (2) |
|---|---|---|---|
| 2,55 % | 1,0 % | > 100 | E/H |
| 1,70 % | 0,67 % | 65 | E/H |
| 1,27 % | 0,50 % | 40 | E/H |
| 0,85 % | 0,33 % | 2,0 | E/H |
| 0,61 % | 0,24 % | 0,4 | H/E |

(1) quantité exprimée en % massique par rapport à la masse totale de l'émulsion
(2) méthode: 1g d'émulsion est dilué dans 5g d'eau déminéralisée. Si l'émulsion est non miscible à l'eau, elle est E/H .

Ces résultats montrent qu'une phase aqueuse dont la viscosité est inférieure à 0,5 Pa.s ne permet pas de réaliser une émulsion eau-dans-huile dans les conditions de l'invention.

**EXEMPLES 8-11 : Préparation d'émulsions solaires selon la présente invention**

Protocoles généraux de mesure des propriétés des émulsions solaires

Détermination du FPS (facteur de protection solaire)

L'indice de protection solaire (IP) ou facteur de protection solaire (FPS) est défini comme étant égal au rapport de la dose érythémale minimale obtenue en utilisant un produit photoprotecteur (DEMp) sur la dose érythémale minimale sans produit (DEMnp), selon le calcul suivant :

$$FPS = DEMp / DEMnp$$

La DEM, exprimée en milliljoules, correspond à la plus petite énergie lumineuse entraînant un érythème perceptible et homogène, à contours nets.

Dans les conditions normales d'un essai pour évaluer le FPS, les volontaires invités au laboratoire lisent une fiche d'information qui leur rappelle les conditions de l'essai avant de signer un formulaire de consentement.

On effectue dans un premier temps des mesures colorimétriques sur les sites à irradier à l'aide d'un appareil tel que le Chromamètre® MINOLTA. En effet, le type de peau doit être déterminée, car le résultat de l'essai est très dépendant de la couleur de la peau du sujet. On répartit les types de peau en six catégories, le type 1 correspondant à la peau la plus blanche rencontrée, qui brûle après une exposition au soleil et qui ne bronze pas du tout. Le type 6 correspond aux teints de peau les plus foncés, les personnes présentant ce phototype (généralement d'origine africaine) possédant une peau qui ne brûle (presque) jamais.

On choisit préférentiellement les sujets de phototypes II et III qui sont les plus représentatifs des phototypes sujets à une augmentation importante de l'érythème par les rayonnements ultra-violets.

Ensuite on applique le produit solaire sur la zone à irradier à raison de 2 mg / $cm^2$. Des produits témoins normalisés par le COLIPA (European Cosmetic Toilettry and Perfumery Association) sont systématiquement testés simultanément et permettent de contrôler la qualité de la manipulation.

Quinze minutes après l'application des produits sur la peau du volontaire, les zones concernées sont irradiées à l'aide d'une lampe au Xénon suivant une progression géométrique de raison 1,25, selon l'indice supposé du produit et de la référence. La lampe au Xénon peut par exemple être une lampe à arc court IDEM 3000® Arquantiel, irradiant sur un spectre allant de 290 à 400 nm. Les rayonnements infrarouges sont filtrés à l'aide d'un filtre de type UG11 (1 mm), et l'élimination des IR est aussi réalisée grâce à un filtre à l'eau et de la ventilation. La surface d'irradiation est d'au moins 1 $cm^2$. La puissance de l'émetteur est d'environ 1000 W.

Un système typique comporte six orifices avec des obturateurs indépendants. L'ouverture successive de chaque orifice à un intervalle de temps fixé par l'opérateur permet d'obtenir une progression géométrique (r = 1,25) de la dose d'UV reçue par le volontaire.

A un moment situé entre 16 et 24 heures après l'irradiation, on réalise la lecture simultanée de la Dose Erythémale Minimale non protégée (DEMnp) et des DEM protégées par la référence et par le produit.

Détermination de la résistance à l'eau des compositions solaires

La rémanence d'un produit solaire est étudiée par irradiation UV après un test de résistance à l'eau. Ce test consiste à faire prendre deux bains aux volontaires à 30 ± 2°C de 20 minutes avec une pause de dix minutes entre chaque bain ("bain standardisé"). Le premier bain est pris 15 minutes après l'application de la composition solaire à tester.

Le pourcentage de rémanence est calculé selon la formule suivante :

$$\%\ de\ rémanence = FPSwr/FPS \times 100$$

avec :

FPS = Indice de protection sec

$FPS_{wr}$ = Indice de protection déterminé après le test de résistance à l'eau

On estime en général que les produits présentant un pourcentage de rémanence inférieur à 50% ne peuvent

pas être caractérisés comme étant résistants à l'eau. Un pourcentage de rémanence compris entre 50% et 80% correspond à un degré acceptable de résistance à l'eau pour un produit solaire. En revanche, les compositions solaires possédant un pourcentage de rémanence supérieur ou égal à 80% (et qui présentent donc une "haute résistance" à l'eau) sont particulièrement recherchées dans le cadre de la présente invention.

Evaluation sensorielle

L'évaluation sensorielle a été réalisée à l'aide d'un panel d'experts (typiquement un jury de 20 personnes), qui note les critères suivants sur une échelle allant de 0 à 10 :

| | | |
|---|---|---|
| Facilité d'étalement | très facile = 10 | très difficile = 0 |
| Sensation de gras | très gras = 10 | absence de sensation de gras = 0 |
| Effet collant | très collant = 10 | absence d'effet collant = 0 |

**Exemple 8 et exemples comparatifs 1 à 3**

Une émulsion solaire ayant la composition indiquée dans le Tableau 4 a été préparée selon la méthode décrite dans l'exemple 1 ci-dessus, les filtres solaires étant incorporés dans la phase grasse. L'indice de photoprotection et le pourcentage de rémanence vis-à-vis de l'eau ont été déterminés suivant les protocoles mentionnés dans la section précédente. Par la suite, l'évaluation sensorielle a été réalisée par le biais d'un panel d'experts.

Parallèlement, des exemples comparatifs ont été réalisés dans des conditions donnant lieu à un gel-crème huile-dans-eau (obtenu sans système émulsionnant), ou à des émulsions constituées d'une phase externe huileuse et d'une phase interne aqueuse non gélifiée.

Les résultats comparatifs sont présentés dans le tableau 4.

Tableau 4

| Exemple | Exemple comparatif 1 | Exemple comparatif 2 | Exemple comparatif 3 | Exemple 8 |
|---|---|---|---|---|
| Nature du produit obtenu | Gel crème H/E | Emulsion E/H | Emulsion E/H | Emulsion E/H à phase aqueuse gélifiée |
| Système émulsionnant | | | | |
| PEG30 dipolyhydroxystéarate<br>Fluidanov® 20X (3) | -<br>- | 0,6%<br>2,4% | 0,6%<br>2,4% | 0,6%<br>2,4% |
| Phase grasse | | | | |
| Caprylic capric triglyceride<br>Cire microcristalline | 12%<br>- | 12%<br>- | 12%<br>5% | 12%<br>- |
| Système filtrant | | | | |
| Oxyde de titane (1)<br>Oxyde de zinc (2)<br>Ethylhexyl salicylate<br>Ethylhexylméthoxycinnamate | 3%<br>3%<br>3,5%<br>5,25% | 3%<br>3%<br>3,5%<br>5,25% | 3%<br>3%<br>3,5%<br>5,25% | 3%<br>3%<br>3,5%<br>5,25% |
| Polymère | | | | |
| SIMULGEL® EG | 3% | - | - | 2,1% |
| Autres additifs | | | | |
| $MgSO_4$ | - | 0,7% | 0,7% | - |

(suite)

| | | | | |
|---|---|---|---|---|
| Autres additifs | | | | |
| Glycérine<br>conservateurs | -<br>qs | 1,5%<br>qs | 1,5%<br>qs | 1,5%<br>qs |
| Eau qsp 100% | | | | |
| Stabilité | granuleux | Instable | Stable | Stable |
| Indice de photoprotection | - | - | 18+/-3 | 18+/-3 |
| % de résistance à l'eau | - | - | 65%+/-10 | 65%+/-10 |
| Evaluation sensorielle | | | | |
| Facilité d'étalement<br>Sensation de gras<br>Effet collant | - | - | 2<br>8<br>8 | 9<br>1<br>0 |
| (1) MT100T SUNSMART<br>(2) Z COTE HP1 TAYCA<br>(3) alkylpolyxyloside sur 2-octyldodécanol, préparé selon EP-A-1142901 | | | | |

On constate que seule l'émulsion selon la présente invention (Exemple 8) allie des performances de résistance à l'eau à une grande facilité d'étalement, un toucher non gras et une absence d'effet collant, avec un indice de photoprotection similaire à celui de l'exemple comparatif 3, qui traduit les connaissances de l'état de la technique de réalisation d'émulsions eau-dans-huile stabilisées par la présence de cires microcristallines et ne mettant pas en oeuvre le procédé selon la présente invention.

**Exemple 9**

L'émulsion solaire est cette fois-ci réalisée avec les composants suivants:

| | |
|---|---|
| 1. MONTANOV® WO18[1] | 1,5% |
| 2. PEG45 dodecyl glycol copolymer | 0,8% |
| 3. Adipate de diisopropyle | 10% |
| 4. TINOSORB M(50%ma)[2] | 20% |
| 5. Méthoxycinnamate d'éthylhexyle | 5% |
| 6. SEPIGEL® 305 | 2% |
| 7. Eau | qsp100% |
| 8. Additifs : conservateurs, parfum, acide citrique | qs |

[1] Isostéaryl glucoside et alcool isostéarylique
[2] Dispersion contenant 50% de matière active

Les ingrédients 1 à 3 sont mélangés et portés à 50°C jusqu'à limpidité. On ajoute les autres ingrédients de la phase grasse 4+5. Séparément on forme un gel avec 6+7. On ajoute la phase grasse au gel.

On obtient au final une émulsion solaire IP30 qui présente un indice de résistance à l'eau de 72%. Cette émulsion est légère, non grasse, non collante et très facile à étaler. Elle présente une quantité de 15% d'huile ; elle est en outre obtenue par un procédé ne nécessitant pas d'apport énergétique important.

L'évaluation sensorielle a donné les notes suivantes :

Facilité d'étalement : 9
Sensation de gras : 1
Effet collant : 0

**Exemple 10 et exemple comparatif 4**

Il s'agit ici de comparer des émulsions selon l'invention, préparée selon le mode opératoire de l'exemple 1,

avec une composition similaire mais non dopée en tensioactifs lipophiles. Ce système de référence communément appelé gel-crème est très répandu sur le marché des soins cosmétiques car il possède des caractéristiques sensorielles recherchées. Il est par contre peu répandu dans les applications solaires en raison de sa faible résistance à l'eau. La reformulation de ces gels-crèmes avec le procédé selon la présente invention, et en faisant intervenir un système tensioactif lipophile, permet de corriger les points faibles et d'obtenir des formulations réunissant performance photoprotectrice, résistance à l'eau et caractéristiques sensorielles agréables.

Tableau 5

| Exemple | Exemple comparatif 4 | Exemple 10 |
|---|---|---|
| Nature du produit obtenu | Gel crème H/E | Emulsion E/H à phase aqueuse gélifiée |
| Système émulsionnant | | |
| PEG30 dipolyhydroxystéarate<br>Fluidanov® 20X | -<br>- | 0,4<br>1,6 |
| Phase grasse | | |
| Di isopropyl adipate | 12% | 12% |
| Système filtrant | | |
| Ethylhexyl salicylate<br>Ethylhexylméthoxycinnamate<br>Ethyl hexyldiméthyl p-aminobenzoate<br>Butylméthoxydibenzoylméthane | 5%<br>5%<br>8%<br>2% | 5%<br>5%<br>8%<br>2% |
| Polymère | | |
| SIMULGEL® NS | 3% | 3% |
| Autres additifs | | |
| Glycérine<br>conservateurs | 1,5%<br>qs | 1,5%<br>qs |
| Eau qsp 100% | | |
| Stabilité | stable | Stable |
| Indice de photoprotection | 15+/-3 | 25+/-3 |
| % de résistance à l'eau | 18%+/-10 | 60%+/-10 |
| Evaluation sensorielle | | |
| Facilité d'étalement<br>Sensation de gras<br>Effet collant | 10<br>2<br>0 | 9<br>2<br>0 |

**<u>Exemple 11 et exemple comparatif 5</u>**

On répète le mode opératoire de l'exemple 10 et de l'exemple comparatif 4, mais avec une phase grasse de structure différente et un système filtrant de nature minérale.

Tableau 6

| Exemple | Exemple comparatif 5 | Exemple 11 |
|---|---|---|
| Nature du produit obtenu | Gel crème H/E | Emulsion E/H à phase aqueuse gélifiée |
| Système émulsionnant | | |
| PEG30 dipolyhydroxystéarate<br>Fluidanov® 20X | -<br>- | 0,6%<br>2,4% |

(suite)

| | | |
|---|---|---|
| Phase grasse | | |
| Caprylic capric triglyceride | 12% | 12% |
| Système filtrant | | |
| Dioxyde de titane uv titan m160 | 10% | 10% |
| Polymère | | |
| SIMULGEL® EG | 2,1% | 2,1% |
| Autres additifs | | |
| Glycérine<br>conservateurs | 1,5%<br>qs | 1,5%<br>qs |
| Eau qsp 100% | | |
| Stabilité | granuleux | Stable |
| Indice de photoprotection | - | 21+/-3 |
| % de résistance à l'eau | - | 51%+/-10 |
| Evaluation sensorielle | - | |
| Facilité d'étalement<br>Sensation de gras<br>Effet collant | | 7<br>3<br>1 |

Le procédé selon l'invention permet d'incorporer un système filtrant de nature minérale avec une étape de gélification de la phase aqueuse, pour atteindre une émulsion eau-dans-huile, alors que la stabilisation s'avère impossible après gélification de la phase aqueuse, dans le schéma huile-dans-eau, sans système tensioactif comme dans la présente invention.

Conclusion générale relative aux exemples 8-11 et exemples comparatifs 1-5

Le procédé de préparation d'émulsions solaires de la présente invention permet :

- D'obtenir des formules photoprotectrices ayant une excellente résistance à l'eau, comparable aux émulsions E/H traditionnelles de l'art antérieur ;
- Avantageusement ces émulsions présentent des caractéristiques sensorielles très intéressantes et similaires aux formulations de type gel-crème. Ces dernières formulations sont très peu présentes dans le domaine solaire car peu compatibles avec les écrans minéraux, peu efficaces en photoprotection, et non résistantes à l'eau.

## Revendications

**1.** Emulsion constituée d'une phase externe grasse et d'une phase aqueuse gélifiée, ladite phase aqueuse représentant 60 à 98 % en poids de la composition, **caractérisée en ce que** :

- la phase aqueuse comprend un polymère de type polyélectrolyte préparé selon un processus de polymérisation en phase inverse sélectionné parmi les éléments du groupe constitué par des homopolymères à base d'un monomère possédant une fonction acide fort, partiellement ou totalement salifiée ; des homopolymères à base d'un monomère possédant une fonction acide faible, partiellement ou totalement salifiée ; des homopolymères à base d'un monomère cationique ; des copolymères à base d'au moins un monomère possédant une fonction acide fort partiellement ou totalement salifiée, copolymérisé soit avec au moins un monomère possédant une fonction acide faible partiellement ou totalement salifiée, soit avec au moins un monomère neutre ; des copolymères à base d'un monomère cationique copolymérisé avec au moins un monomère neutre ; des copolymères à base d'au moins un monomère possédant une fonction acide faible partiellement ou totalement salifiée copolymérisé soit avec au moins un monomère possédant une fonction acide faible partiellement ou totalement

salifiée, soit avec au moins un monomère neutre; et
- la phase grasse comprend une ou plusieurs huiles et un système émulsionnant comprenant un ou plusieurs tensioactifs émulsionnants, sélectionnés parmi les éléments du groupe constitué des alkylpolyglycosides, des compositions d'alkylpolyglycoside(s) et d'alcool(s) gras, des esters de polyglycérols éventuellement alcoxylés, des polyhydroxystéarates de polyols (tels que les polyglycols ou les polyglycérols) éventuellement alcoxylés, des copolymères polyéthylèneglycol-alkylglycols

**2.** Emulsion selon la revendication 1, qui est une émulsion solaire, dont la phase huileuse et/ou la phase aqueuse comprend (comprennent) un ou plusieurs filtres solaires.

**3.** Emulsion solaire selon la revendication 2, dans laquelle le système émulsionnant comprend un ester de polyglycérol éventuellement alcoxylé, un polyhydroxystéarate de polyglycol éventuellement alcoxylé, ou un copolymère polyéthylèneglycol-alkylglycol en combinaison avec un alkylpolyglycoside ou une composition d'alkylpolyglycoside(s) et d'alcool(s) gras.

**4.** Emulsion solaire selon l'une quelconque des revendications 2 ou 3, dans laquelle le ou les filtres solaires représentent environ 2% à environ 40%, de préférence environ 5% à environ 20% en poids de l'émulsion.

**5.** Emulsion solaire selon l'une des revendications 2 à 4, dans laquelle la phase huileuse comprend en outre une ou plusieurs charges minérales.

**6.** Procédé de préparation d'une émulsion de type eau dans huile, telle que définie à l'une des revendications 1 ou 2, comprenant les étapes suivantes :

a) on prépare une phase grasse comprenant une ou plusieurs huiles, et un système émulsionnant comprenant un ou plusieurs tensioactifs émulsionnants, sélectionnés parmi les éléments du groupe constitué des alkylpolyglycosides, des compositions d'alkylpolyglycoside(s) et d'alcool(s) gras, des esters de polyglycérols éventuellement alcoxylés, des polyhydroxystéarates de polyols (tels que les polyglycols ou les polyglycérols) éventuellement alcoxylés, des copolymères polyéthylèneglycol-alkylglycols et éventuellement un ou plusieurs filtres solaires;
b) on prépare, indépendamment de la phase grasse, une phase aqueuse gélifiée contenant un polymère de type polyélectrolyte préparé selon un processus de polymérisation en phase inverse sélectionné parmi les éléments du groupe constitué par des homopolymères à base d'un monomère possédant une fonction acide fort, partiellement ou totalement salifiée ; des homopolymères à base d'un monomère possédant une fonction acide faible, partiellement ou totalement salifiée ; des homopolymères à base d'un monomère cationique ; des copolymères à base d'au moins un monomère possédant une fonction acide fort partiellement ou totalement salifiée, copolymérisé soit avec au moins un monomère possédant une fonction acide faible partiellement ou totalement salifiée, soit avec au moins un monomère neutre ; des copolymères à base d'un monomère cationique copolymérisé avec au moins un monomère neutre ; des copolymères à base d'au moins un monomère possédant une fonction acide faible partiellement ou totalement salifiée copolymérisé soit avec au moins un monomère possédant une fonction acide faible partiellement ou totalement salifiée, soit avec au moins un monomère neutre, et éventuellement un ou plusieurs filtres solaires;
c) on ajoute la phase grasse sur la phase aqueuse.

**7.** Procédé selon la revendication 6, dans lequel le système émulsionnant comprend ester de polyglycérol éventuellement alcoxylé, un polyhydroxystéarate de polyglycol éventuellement alcoxylé, ou un copolymère polyéthylèneglycol-alkylglycol, en combinaison avec un alkylpolyglycoside ou une composition d'alkylpolyglycoside(s) et d'alcool (s) gras.

**8.** Procédé selon l'une quelconque des revendications 6 et 7 dans lequel ledit polymère de type polyélectrolyte est choisi parmi le groupe constitué de copolymères ou d'homopolymères, qui peuvent ou non être réticulés ou ramifiés, à base de monomères possédant une fonction acide fort ou acide faible partiellement ou totalement salifiée, ou une fonction cationique, lesdits monomères étant de préférence choisis parmi l'acide styrène sulfonique ou le méthacrylate de 2-sulfoéthyle, l'acide styrène phosphonique, partiellement ou totalement salifié, l'acide-2-méthyl-[(1-oxo-2-propényl)amino]1-propane sulfonique (AMPS) partiellement ou totalement salifié sous forme de sel de sodium, de sel d'ammonium ou de sel de monoéthanolamine.

**9.** Procédé selon l'une quelconque des revendications 6 à 8, dans lequel le polymère de type polyélectrolyte est choisi

parmi les copolymères de l'acide acrylique et de l'acide-2-méthyl-[(1-oxo-2-propényl)amino] 1-propane sulfonique (AMPS), les copolymères de l'acrylamide et de l'acide-2-méthyl-[(1-oxo-2-propényl)amino] 1-propane sulfonique, les copolymères de l'acide-2-méthyl-[(1-oxo-2-propényl)amino] 1-propane sulfonique et de l'acrylate de (2-hydroxyéthyle), l'homopolymère de l'acide-2-méthyl-[(1-oxo-2-propényl)amino] 1-propane sulfonique, l'homopolymère de l'acide acrylique, les copolymères du chlorure d'acryloyl éthyl triméthyl ammonium et de l'acrylamide, les copolymères de l'AMPS et de la vinylpyrrolidone, les copolymères de l'acide acrylique et d'acrylates d'alkyle dont la chaîne carbonée comprend entre dix et trente atomes de carbone, les copolymères de l'AMPS et d'acrylates d'alkyle dont la chaîne carbonée comprend entre dix et trente atomes de carbone

**10.** Procédé selon l'une quelconque des revendications 6 à 9, dans lequel la phase aqueuse comprend au moins un tensioactif émulsionnant.

**11.** Procédé selon l'une des revendications 6 à 10, dans lequel la phase aqueuse gélifiée est obtenue par mise en solution dudit polymère de type polyélectrolyte et présente une viscosité comprise entre 0,5 et 300 Pa.s, préférentiellement entre 1,0 et 150 Pa.s et plus particulièrement entre 5 et 100 Pa.s.

**12.** Procédé selon l'une quelconque des revendications 6 à 11 dans lequel la phase grasse est ajoutée sur la phase aqueuse à une température inférieure à 55°C et de préférence comprise entre 15 et 35°C.

**13.** Procédé selon l'une quelconque des revendications 6 à 12 dans lequel les deux phases sont mélangées avec une vitesse d'agitation inférieure à 1000 tours par minute et de préférence comprise entre 80 et 800 tours par minute.

**14.** Préparation pharmaceutique, vétérinaire ou détergente comprenant une émulsion selon la revendication 1 ou une émulsion préparée par le procédé selon l'une quelconque des revendications 6 à 13.

**15.** Préparation cosmétique comprenant une émulsion selon l'une quelconque des revendications 1 à 5.

**Patentansprüche**

**1.** Emulsion, die aus einer äußeren Fettphase und einer gelierten Wasserphase gebildet ist, wobei die Wasserphase 60 bis 98 Gewichts-% der Zusammensetzung darstellt, **dadurch gekennzeichnet, dass**:

- die Wasserphase ein in einem inversen Polymerisationsverfahren hergestelltes Polymer vom Typ Polyelektrolyt umfasst, das ausgewählt ist aus den Elementen der Gruppe bestehend aus Homopolymeren auf der Grundlage eines Monomers, das eine stark saure funktionelle Gruppe besitzt, die teilweise oder vollständig in ein Salz umgewandelt ist; Homopolymeren auf der Grundlage eines Monomers, das eine schwach saure funktionelle Gruppe besitzt, die teilweise oder vollständig in ein Salz umgewandelt ist; Homopolymeren auf der Grundlage eines kationischen Monomers; Copolymeren auf der Grundlage von mindestens einem Monomer, das eine stark saure funktionelle Gruppe besitzt, die teilweise oder vollständig in ein Salz umgewandelt ist, und entweder mit einem Monomer copolymerisiert ist, das eine schwach saure funktionelle Gruppe besitzt, die teilweise oder vollständig in ein Salz umgewandelt ist, oder mit mindestens einem neutralen Monomer; Copolymeren auf der Grundlage von einem kationischen Monomer, das mit mindestens einem neutralen Monomer copolymerisiert ist; Copolymeren auf der Grundlage von mindestens einem Monomer, das eine schwach saure funktionelle Gruppe besitzt, die teilweise oder vollständig in ein Salz umgewandelt ist, und entweder mit einem Monomer copolymerisiert ist, das eine schwach saure funktionelle Gruppe besitzt, die teilweise oder vollständig in ein Salz umgewandelt ist, oder mit mindestens einem neutralen Monomer; und
- die Fettphase ein oder mehrere Öle umfasst und ein Emulgierungssystem, das ein oder mehrere emulgierende Tenside umfasst, die ausgewählt sind aus den Elementen der Gruppe bestehend aus Alkylpolyglykosiden, Zusammensetzungen aus Alkylpolyglykosid(en) und Fettalkohol(en), Estern von möglicherweise alkoxylierten Polyglycerinen, Polyhydroxystearaten von möglicherweise alkoxylierten Polyolen (wie Polyglykolen oder Polyglycerinen), Polyethylenglykol-Alkylglykol-Copolymeren.

**2.** Emulsion nach Anspruch 1, die eine Sonnenschutz-Emulsion ist, deren Ölphase und/oder Wasserphase einen oder mehrere Sonnenschutzfilter umfasst (umfassen).

**3.** Sonnenschutz-Emulsion nach Anspruch 2, bei der das Emulgierungssystem ein Ester eines möglicherweise alkoxylierten Polyglycerins, ein Polyhydroxystearat eines möglicherweise alkoxylierten Polyglykols oder ein Polyethylenglykol-Alkylglykol-Copolymer in Kombination mit einem Alkylpolyglykosid oder einer Zusammensetzung aus Alkylpolyglykosid(en) und Fettalkohol(en) umfasst.

**4.** Sonnenschutz-Emulsion nach einem der Ansprüche 2 oder 3, in der der oder die Sonnenschutzfilter ungefähr 2% bis ungefähr 40%, vorzugsweise ungefähr 5% bis ungefähr 20% des Gewichts der Emulsion darstellen.

**5.** Sonnenschutz-Emulsion nach einem der Ansprüche 2 bis 4, in der die Ölphase ferner einen oder mehrere mineralische Füllstoffe umfasst.

**6.** Verfahren zur Herstellung einer Wasser-in-Öl-Emulsion nach einem der Ansprüche 1 oder 2, das folgende Schritte umfasst:

a) Herstellen einer Fettphase, die ein oder mehrere Öle umfasst und ein Emulgierungssystem, das ein oder mehrere emulgierende Tenside umfasst, die ausgewählt werden aus den Elementen der Gruppe bestehend aus Alkylpolyglykosiden, Zusammensetzungen aus Alkylpolyglykosid(en) und Fettalkohol(en), Estern von möglicherweise alkoxylierten Polyglycerinen, Polyhydroxystearaten von möglicherweise alkoxylierten Polyolen (wie Polyglykolen oder Polyglycerinen), Polyethylenglykol-Alkylglykol-Copolymeren und möglicherweise einen oder mehrere Sonnenschutzfilter;
b) unabhängig von der Fettphase Herstellen einer gelierten Wasserphase, die ein in einem inversen Polymerisationsverfahren hergestelltes Polymer vom Typ Polyelektrolyt enthält, das ausgewählt wird aus den Elementen der Gruppe bestehend aus Homopolymeren auf der Grundlage eines Monomers, das eine stark saure funktionelle Gruppe besitzt, die teilweise oder vollständig in ein Salz umgewandelt ist; Homopolymeren auf der Grundlage eines Monomers, das eine schwach saure funktionelle Gruppe besitzt, die teilweise oder vollständig in ein Salz umgewandelt ist; Homopolymeren auf der Grundlage eines kationischen Monomers; Copolymeren auf der Grundlage von mindestens einem Monomer, das eine stark saure funktionelle Gruppe besitzt, die teilweise oder vollständig in ein Salz umgewandelt ist, und entweder mit einem Monomer copolymerisiert ist, das eine schwach saure funktionelle Gruppe besitzt, die teilweise oder vollständig in ein Salz umgewandelt ist, oder mit mindestens einem neutralen Monomer; Copolymeren auf der Grundlage von einem kationischen Monomer, das mit mindestens einem neutralen Monomer copolymerisiert ist; Copolymeren auf der Grundlage von mindestens einem Monomer, das eine schwach saure funktionelle Gruppe besitzt, die teilweise oder vollständig in ein Salz umgewandelt ist, und entweder mit einem Monomer copolymerisiert ist, das eine schwach saure funktionelle Gruppe besitzt, die teilweise oder vollständig in ein Salz umgewandelt ist, oder mit mindestens einem neutralen Monomer; und möglicherweise einen oder mehrere Sonnenschutzfilter;
c) Zugeben der Fettphase zur Wasserphase.

**7.** Verfahren nach Anspruch 6, wobei das Emulgierungssystem ein Ester eines möglicherweise alkoxylierten Polyglycerins, ein Polyhydroxystearat eines möglicherweise alkoxylierten Polyglykols oder ein Polyethylenglykol-Alkylglykol-Copolymer in Kombination mit einem Alkylpolyglykosid oder einer Zusammensetzung aus Alkylpolyglykosid(en) und Fettalkohol(en) umfasst.

**8.** Verfahren nach einem der Ansprüche 6 und 7, wobei das Polymer vom Typ Polyelektrolyt ausgewählt wird aus der Gruppe bestehend aus Copolymeren oder Homopolymeren, die vernetzt oder verzweigt oder nicht vernetzt oder nicht verzweigt sein können, auf der Grundlage von Monomeren, die eine stark saure oder schwach saure funktionelle Gruppe besitzen, die teilweise oder vollständig in ein Salz umgewandelt ist, oder eine kationische funktionelle Gruppe, wobei die Monomere vorzugsweise ausgewählt sind aus Styrolsulfonsäure oder 2-Sulfoethylmethacrylat, Styrolphosphonsäure, teilweise oder vollständig in ein Salz umgewandelt, 2-Methyl-[(1-oxo-2-propenyl)amino]-1-propansulfonsäure (AMPS), teilweise oder vollständig in ein Salz in Form des Natriumsalzes, Ammoniumsalzes oder Monoethanolaminsalzes umgewandelt.

**9.** Verfahren nach einem der Ansprüche 6 bis 8, wobei das Polymer vom Typ Polyelektrolyt ausgewählt wird aus Copolymeren aus Acrylsäure und 2-Methyl-[(1-oxo-2-propenyl)amino]-1-propansulfonsäure (AMPS), Copolymeren aus Acrylamid und 2-Methyl-[(1-oxo-2-propenyl)amino]-1-propansulfonsäure, Copolymeren aus 2-Methyl-[(1-oxo-2-propenyl)amino]-1-propansulfonsäure und 2-Hydroxyethylacrylat, dem Homopolymer von 2-Methyl-[(1-oxo-2-propenyl)amino]-1-propansulfonsäure, dem Homopolymer von Acrylsäure, Copolymeren aus Acryloyl-ethyl-trimethylammoniumchlorid und Acrylamid, Copolymeren aus AMPS und Vinylpyrrolidon, Copolymeren aus Acrylsäure und Alkylacrylaten, deren Kohlenstoffkette zwischen zehn und dreißig Kohlenstoffatome umfasst, Copolymeren aus AMPS und Alkylacrylaten, deren Kohlenstoffkette zwischen zehn und dreißig Kohlenstoffatome umfasst.

10. Verfahren nach einem der Ansprüche 6 bis 9, wobei die Wasserphase mindestens ein emulgierendes Tensid umfasst.

11. Verfahren nach einem der Ansprüche 6 bis 10, wobei die gelierte Wasserphase durch Lösen des Polymers vom Typ Polyelektrolyt erhalten wird und eine Viskosität zwischen 0,5 und 300 Pa.s, vorzugsweise zwischen 1,0 und 150 Pa.s und insbesondere zwischen 5 und 100 Pa.s aufweist.

12. Verfahren nach einem der Ansprüche 6 bis 11, wobei die Fettphase bei einer Temperatur von unter 55°C und vorzugsweise zwischen 15 und 35°C zur Wasserphase gegeben wird.

13. Verfahren nach einem der Ansprüche 6 bis 12, wobei die beiden Phasen mit einer Rührgeschwindigkeit von unter 1000 Umdrehungen pro Minute und vorzugsweise zwischen 80 und 800 Umdrehungen pro Minute vermischt werden.

14. Pharmazeutisches, tiermedizinisches oder reinigendes Präparat, das eine Emulsion nach Anspruch 1 oder eine Emulsion umfasst, die mit dem Verfahren nach einem der Ansprüche 6 bis 13 hergestellt ist.

15. Kosmetisches Präparat, das eine Emulsion nach einem der Ansprüche 1 bis 5 umfasst.

**Claims**

1. Emulsion composed of a fatty external phase and of a gelled aqueous phase, said aqueous phase representing 60% to 98% by weight of the composition, **characterised in that**:

   - the aqueous phase comprises a polyelectrolyte polymer prepared by a reverse-phase polymerisation process selected from the members of the group consisting of homopolymers based on a monomer having a strong acid functional group which is partially or completely salified; homopolymers based on a monomer having a weak acid functional group which is partially or completely salified; homopolymers based on a cationic monomer; copolymers based on at least one monomer having a strong acid functional group which is partially or completely salified, which monomer is copolymerised, either with at least one monomer having a weak acid functional group which is partially or completely salified, or with at least one neutral monomer; copolymers based on a cationic monomer copolymerised with at least one neutral monomer; copolymers based on at least one monomer having a weak acid functional group which is partially or completely salified, which monomer is copolymerised, either with at least one monomer having a weak acid functional group which is partially or completely salified, or with at least one neutral monomer; and
   - the fatty phase comprises one or more oils and an emulsifying system comprising one or more emulsifying surfactants, selected from the members of the group consisting of alkylpolyglycosides, compositions formed of alkylpolyglycoside(s) and of fatty alcohol(s), optionally alkoxylated polyglycerol esters, optionally alkoxylated polyol (such as polyglycols or polyglycerols) polyhydroxystearates, polyethylene glycol/alkyl glycol copolymers.

2. Emulsion according to claim 1, which is a sun-protection emulsion, in which the oily phase and/or the aqueous phase comprise(s) one or more sunscreens.

3. Sun-protection emulsion according to claim 2, wherein the emulsifying system comprises an optionally alkoxylated polyglycerol ester, an optionally alkoxylated polyglycol polyhydroxystearate, or a polyethylene glycol/alkyl glycol copolymer in combination with an alkylpolyglycoside or a composition formed of alkylpolyglycoside(s) and of fatty alcohol(s).

4. Sun-protection emulsion according to either claim 2 or claim 3, wherein the sunscreen(s) represent approximately 2% to approximately 40%, preferably approximately 5% to approximately 20% by weight of the emulsion.

5. Sun-protection emulsion according to any of claims 2 to 4, wherein the oily phase further comprises one or more inorganic fillers.

6. Process for preparing a water-in-oil emulsion, as defined in either claim 1 or claim 2, comprising the following steps:

   a) preparing a fatty phase comprising one or more oils, and an emulsifying system comprising one or more emulsifying surfactants, selected from the members of the group consisting of alkylpolyglycosides, compositions formed of alkylpolyglycoside(s) and of fatty alcohol(s), optionally alkoxylated polyglycerol esters, optionally

alkoxylated polyol (such as polyglycols or polyglycerols) polyhydroxystearates, polyethylene glycol/alkyl glycol copolymers and optionally one or more sunscreens;

b) preparing, independently of the fatty phase, a gelled aqueous phase containing a polyelectrolyte polymer prepared by a reverse-phase polymerisation process selected from the members of the group consisting of homopolymers based on a monomer having a strong acid functional group which is partially or completely salified; homopolymers based on a monomer having a weak acid functional group which is partially or completely salified; homopolymers based on a cationic monomer; copolymers based on at least one monomer having a strong acid functional group which is partially or completely salified, which monomer is copolymerised, either with at least one monomer having a weak acid functional group which is partially or completely salified, or with at least one neutral monomer; copolymers based on a cationic monomer copolymerised with at least one neutral monomer; copolymers based on at least one monomer having a weak acid functional group which is partially or completely salified, which monomer is copolymerised, either with at least one monomer having a weak acid functional group which is partially or completely salified, or with at least one neutral monomer, and optionally one or more sunscreens;

c) adding the fatty phase to the aqueous phase.

**7.** Process according to claim 6, wherein the emulsifying system comprises optionally alkoxylated polyglycerol ester, an optionally alkoxylated polyglycol polyhydroxystearate, or a polyethylene glycol/alkyl glycol copolymer, in combination with an alkylpolyglycoside or a composition formed of alkylpolyglycoside(s) and of fatty alcohol(s).

**8.** Process according to either claim 6 or claim 7, wherein said polyelectrolyte polymer is selected from the group consisting of copolymers or homopolymers, which may or may not be crosslinked or branched, based on monomers having a partially or completely salified strong acid or weak acid functional group, or a cationic functional group, said monomers preferably being selected from styrenesulphonic acid or 2-sulphoethyl methacrylate, styrenephosphonic acid which is partially or completely salified, 2-methyl-[(1-oxo-2-propenyl)amino]-1-propanesulphonic acid (AMPS) which is partially or completely salified in the form of sodium salt, of ammonium salt, or of monoethanolamine salt.

**9.** Process according to any of claims 6 to 8, wherein the polyelectrolyte polymer is selected from copolymers of acrylic acid and of 2-methyl-[(1-oxo-2-propenyl)amino]-1-propanesulphonic acid (AMPS), copolymers of acrylamide and of 2-methyl-[(1-oxo-2-propenyl)amino]-1-propanesulphonic acid, copolymers of 2-methyl-[(1-oxo-2-propenyl)amino]-1-propanesulphonic acid and of 2-hydroxyethyl acrylate, 2-methyl-[(1-oxo-2-propenyl)amino]-1-propanesulphonic acid homopolymer, acrylic acid homopolymer, copolymers of acryloylethyltrimethylammonium chloride and of acrylamide, copolymers of AMPS and of vinylpyrrolidone, copolymers of acrylic acid and of alkyl acrylates, in which the carbonaceous chain comprises between ten and thirty carbon atoms, copolymers of AMPS and of alkyl acrylates, in which the carbonaceous chain comprises between ten and thirty carbon atoms.

**10.** Process according to any of claims 6 to 9, wherein the aqueous phase comprises at least one emulsifying surfactant.

**11.** Process according to any of claims 6 to 10, wherein the gelled aqueous phase is obtained by dissolving said polyelectrolyte polymer and has a viscosity of between 0.5 and 300 Pa.s, preferably of between 1.0 and 150 Pa.s and more particularly of between 5 and 100 Pa.s.

**12.** Process according to any of claims 6 to 11, wherein the fatty phase is added to the aqueous phase at a temperature of less than 55 °C and preferably of between 15 °C and 35 °C.

**13.** Process according to any of claims 6 to 12, wherein the two phases are mixed with a stirring rate of less than 1000 revolutions per minute and preferably of between 80 and 800 revolutions per minute.

**14.** Pharmaceutical, veterinary or detergent preparation comprising an emulsion according to claim 1 or an emulsion prepared by the process according to any of claims 6 to 13.

**15.** Cosmetic preparation comprising an emulsion according to any of claims 1 to 5.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 5746945 A
- WO 9740814 A
- WO 02100374 A
- EP 0503853 A
- FR 2816836
- EP 1325729 A
- EP 1243252 A
- EP 1166770 A
- EP 1000542 A
- WO 9600719 A
- FR 2790977 A
- EP 1142901 A